(19) Europäisches Patentamt
European Patent Office
Office européen des brevets

(11) Publication number: **0 292 050 B1**

# (12) EUROPEAN PATENT SPECIFICATION

(45) Date of publication of patent specification: **21.11.91 Bulletin 91/47**

(51) Int. Cl.$^5$: **A61K 47/08**, A61K 9/18, A61K 31/495

(21) Application number: **88200931.9**

(22) Date of filing: **10.05.88**

(54) **Improved flunarizine-containing compositions.**

(30) Priority: **18.05.87 US 50644**

(43) Date of publication of application: **23.11.88 Bulletin 88/47**

(45) Publication of the grant of the patent: **21.11.91 Bulletin 91/47**

(84) Designated Contracting States: **AT BE CH DE ES FR GB GR IT LI LU NL SE**

(56) References cited:
**EP-A- 0 149 197**
**EP-A- 0 151 987**
**EP-A- 0 167 825**
**EP-A- 0 197 571**

(73) Proprietor: **JANSSEN PHARMACEUTICA N.V.**
**Turnhoutseweg 30**
**B-2340 Beerse (BE)**

(72) Inventor: **Mesens, Jean**
**Moereind 17**
**D-2152-Wechelderzande (BE)**
Inventor: **Van Peer, Achiel Paul**
**Dennedreef 13**
**B-2350-Vosselaar (BE)**
Inventor: **Heykants, Jozef Jan Pieter**
**Karel Druytslaan 9**
**B-2350-Vosselaar (BE)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

### Background of the invention

The most critical factor for an effective oral therapy is most probably the rate and extent of gastro-intestinal absorption of the drug. Failure of therapy or side-effects are often related to malabsorption. Besides the physico-chemical properties of the drug, systemic drug availability can be influenced by a lot different physiological factors. Gastro-intestinal contents and pH, gastric emptying, intestinal transit time and metabolism, drug-food and drug-drug interactions, disease states and physiologic disorders are critical parameters in the process of drug-absorption.

It is known that changes in gastric pH as a result of physiological causes or by neutralization by anti-acids or inhibition of gastric secretion by $H_2$-antagonists, can significantly decrease the absorption of drugs. Especially weak basic substances require an acidic environment for optimal dissolution and consequently absorption of these substances is often inhibited. In such instances one can expect differences in oral bioavailability where gastric pH is increased and this phenomenon can have a serious impact on the efficacy of the drug.

From the above it is clear that an effective therapy for such substances is highly dependent upon favourable gastro-intestinal pH conditions. The ultimate goal to improve the therapy for such substances could be achieved by making absorption of the drug independent of pH.

It now has been found that the bioavailability of the active ingredient (E)-1-[bis(4-fluorophenyl)methyl]-4-(3-phenyl-2-propenyl]piperazine, which compound generically is designated as flunarizine, can be increased and stabilized and more particularly be made independent of pH-fluctuations by formulating this compound in compositions containing particular surfactants, especially in high concentrations, or particular cyclodextrins or derivatives thereof as carriers.

Flunarizine is a weak base, is quite lipophilic and is poorly-soluble in neutral or quasi-neutral aqueous mediums. An increase of the gastric pH will undoubtly negatively influence the oral bioavailability of this compound and a certain inpact on its efficacy may be expected. Since flunarizine is mainly indicated in conditions of impaired cerebro-vascular and peripheral blood flow its target population are mainly elderly patients. Often this group of patients have a high incidence of gastro-intestinal disorders as achlorhydrie, reflux-oesophagitis, gastric and duodenal ulcers and gastritis which affect normal gastric secretion. Moreover, since flunarizine medication is often taken at the evening, reduced nocturnal gastric secretion could even more aggravate the unfavourable conditions.

The present invention is concerned with pharmaceutical compositions containing polyethoxylated castor oil or hydrogenated castor oil obtained by reaction of 1 mole of the said oil with 5 to 100 moles of ethylene oxide, and as active ingredient a chemical compound having the formula

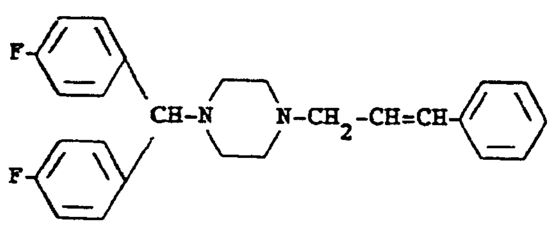

(I),

a pharmaceutically acceptable acid-addition salt thereof, or a stereochemically isomeric form thereof.

The said compounds of formula (I), as well as methods for preparing the same are described in U.S. Patent No. 3,773,939.

The compound of formula (I) wherein the substituents on the double bond are in a (E)-configuration is generically designated as flunarizine.

This compound is now widely used as an anti-histaminic and in the treatment of cerebral and/or peripheral vascular insufficiency, of vertigo and motion sickness.

In the compositions according to the present invention, the compound of formula (I) may be used as such or as an acid-addition salt form. The latter can be obtained by reacting the free-base form with an appropriate acid, such as, for example, inorganic acids, such as an hydrohalic acid, e.g. hydrochloric acid, and hydrobromic, and sulfuric acid, nitric acid, and phosphoric acid or organic acids, such as, for example, acetic, propanoic, hydroxyacetic, 2-hydroxypropanoic, 2-oxopropanoic, ethanedioic, propanedioic, butanedioic, (Z)-2-butenedioic, (E)-2-butenedioic, 2-hydroxybutanedioic, 2,3-dihydroxybutanedioic, 2-hydroxy-1,2,3-propanetricarboxylic, mathanesulfonic, ethanesulfonic, benzenesulfonic, 4-methylbenzenesulfonic, cyclohexanesulfamic,

2-hydroxybenzoic, and 4-amino-2-hydroxybenzoic acids.

As used throughout the description of the present invention, the term "flunarizine" is meant to comprise the compound (E)-1-[bis(4-fluorophenyl)methyl] -4-(3-phenyl-2-propenyl]piperazine as well as the pharmaceutically acceptable acid-addition salts thereof.

A particularly preferred subgroup of carriers comprises those polyethoxylated castor or hydrogenated castor oils obtained by reaction of 1 mole of the said oil with 5 to 100 moles, in particular with 10 to 80 moles, more particularly with 20 to 60 moles and preferably with 30 to 50 moles, and more preferably with 35 or with 40 moles of ethylene oxide. Commercially available polyethoxylated castor or hydrogenated castor oils are sold under the trade-narks Cremophor EL® or Cremophor RH® ; Cremophor EL® being the reaction product of castor oil with ethylene oxide, and Cremophor RH® being the reaction product of hydrogenated castor oil with ethylene oxide, whereby are most preferred, those polyethoxylated castor oils or hydrogenated castor oils obtained by the reaction of 1 mole of cantor oil with 40 mole of ethylene oxide (indicated as Cremophor EL®) or of 1 mole of hydrogenated castor oil with 40 moles of ethylene oxide (usually indicated as Cremophor RH-40®). Such Cremophor® products are available from B.A.S.F., Ludwigshaven, Germany.

To prepare the pharmaceutical compositions of the present invention the active ingredient is combined in intimate admixture with the aforementioned special carrier and, if desired, other adjuvants. The latter may take a variety of forms depending on the form of preparation desired for administration.

The pharmaceutical compositions of the present invention are desirably in unitary dosage form suitable for administering orally, rectally or by parenteral injection, the oral route of administration being preferred.

As examples of compositions in oral dosage form, there may be mentioned oral liquid preparations such as suspensions, syrups, elixirs and solutions ; or powders, pills, capsules and tablets ; as examples of parenteral compositions there may be mentioned injectable solutions and as examples of rectally applicable compositions, suppositories. Because of their ease of administration, tablets and capsules represent the most advantageous oral dosage unit form.

In case of compostions in oral dosage form, the aforementioned adjuvants may take a wide variety of forms. For oral liquid preparations they comprise e.g. water, glycols, oils, and alcohols, whereas for solid preparations starches, sugars, kaolin, lubricants, binders, thickeners, and desintegrating agents are most conveniently employed. For parenteral compositions said adjuvants comprise sterile water, saline solution, glucose solution or a mixture of saline and glucose solution.

It is evident that the special carrier for use in the compositions according to the present invention will have to be selected in function of the kind of the composition desired for administration. For solid formulations such as capsules, tablets, pills or powders the special carrier will preferably have to be solid, or if liquid or semi-liquid special carrier is used, an appropriate adjuvant such as a binder or a thickener will have to be added.

It is especially advantageous to formulate the aforementioned pharmaceutical compositions in dosage unit form for ease of administration and uniformity of dosage. Dosage unit form as herein referred to, refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoon-fuls, and segregated multiples thereof.

The compositions of the present invention are most conveniently prepared by warming the carrier untill it becomes liquid or semi-liquid and to concomitantly or subsequently throroughly mix said carrier with the active ingredient. The thus obtained mixture is then allowed to cool. Or, if desired, the warm mixture is filled into appropriate capsules, in particular hard gelatine capsules, which are sealed. In this way the active ingredient is finely dispersed in the carrier, thus, depending on the degree of dispersion, forming a so-called solid dispersion or a solid solution.

Compared with the art-known pharmaceutical compositions containing a compound of formula (I) as active ingredient, the present compositions show the advantage that the bioavailability of the active substance is increased. As a result thereof a smaller amount of the active ingredient needs to be administered in order to obtain the desired therapeutic effect. Furthermore, when administering the compositions of the present invention, there is less incertainty of the actual plasma levels of the active ingredient after administration of a certain amount of the active ingredient, whereas the obtained plasma levels are less dependent on physicochemical and/or physiological parameters. More particularly the influence of the pH fluctuations in the gastro-intestinal tract and especially of the gastric juices on the bioavailability are reduced or even eliminated when using the compositions of the present invention. The increased and improved bioavailability is quite unexpected as it is known that surfactants often inhibit absorption of the drug and thus reduce its bioavailibility due to micellar formation.

In a further aspect of the present invention there are provided compositions, preferably for use in capsules,

which compositions contain a compound of formula (I) as defined hereinabove, as active ingredient, and as carrier a polyethoxylated castor or hydrogenated castor oil as defined above and, if desired other adjuvants, whereby the said surfactant makes up the major part of the composition. Preferably, the said compositions contain over 50% and more preferably over 80% of the said castor oil. Particularly preferred compositions are those compositions containing over 80% of a polyethoxylated castor oil or hydrogenated castor oil as a carrier and further containing form 0 to 10% and preferably form 0 to 5% of other adjutants, the latter preferably comprising water. Such other adjuvants may comprise, besides water, suitable bases, e.g. alkali metal or earth alkaline metal carbonates, hydrogen carbonates or hydroxides such as calcium carbonate, potassium, sodium or calcium hydroxide ; or any of the aforementioned other adjuvants such as glycols, oils, alcohols, starches, sugars, kaolin, lubricants, binders, thickeners, and desintegrating agents.

One form of the invention comprises a hard gelatine capsule containing a composition containing flunarizine as active ingredient, over 80% of a Cremophor® as defined hereinabove and a small amount of water said amount of water ranging from 0.1 to 5% by weight, particularly from 0.1 to 2.5% by weight, and optionally a small amount of an alkali or earth alkaline metal hydroxide or carbonate, in particular sodium hydroxide, said amount of said base and in particular of sodium hydroxide ranging from 0.1 to 3% and particularly from 0.1 to 1.5% by height. The unit dose of flunarizine, expressed as base equivalent, in the said capsule is in the range of 1 to 10 mg and in particular in the range of 2 to 8 mg. Typical unit doses of flunarizine, espressed as base equivalent, are 3.5 mg, 5 mg or 7mg.

The presence of such an excess of the said surfactant resulting in compostions showing the aforementioned useful pharmacological effects is quite unexpected since it is known that the activity of a drug is decreased when surfactants are employed in high concentrations.

Examples.

As used in the following examples Cremophor RH40® is a trademark for ethoxylated hydrogenated cantor oil obtained by treating 1 mole of hydrogenated castor oil with about 40 moles of ethylene oxide. Cremophor EL® is a trademark for ethoxylated castor oil obtained by treating 1 mole of castor oil with about 40 moles of ethylene oxide. Aerosil® is a trademark for colloidal $SiO_2$ ; Pluronic F68/F35® for a block-copolymer containing hydrophylic polyoxyethylene and hydrophobic polypropylene blocks.

A. Composition examples

Example 1 (reference formulation)

| | |
|---|---|
| Flunarizine hydrochloride | 5.9 mg |
| Lactose extra fine crystals | 61.4 mg |
| Lactose | 61.3 mg |
| Starch | 20.0 mg |
| Talcum | 9 mg |
| Magnesium stearate | 2 mg |
| Aerosil® | 0.4 mg |

The ingredients were thouroughly mixed and filled into capsules

Example 2

| | |
|---|---|
| Flunarizine dihydrochloride | 4.13 mg |
| Sodium hydroxide | 0.692 mg |
| Dist. water | 1.058 mg |
| Cremophor RH40® | 94.12 mg |

The ingredients are thoroughly mixed in a heat-jacketed vessel at 45°C-50°C. At this temperature the mixture is filled into hard gelatine capsules and sealed using appropriate equipment.

Example 3

| | |
|---|---|
| Flunarizine dihydrochloride | 4.13 mg |
| sodium hydroxide | 0.692 mg |
| dist. water | 1.058 mg |
| Cremophor RH 40® | 144.12 mg |

Example 4

| | |
|---|---|
| Flunarizine dihydrochloride | 8.26 mg |
| sodium hydroxide | 1.384 mg |
| dist. water | 2.116 mg |
| Cremophor RH 40® | 138.240 mg |

Same manufacture procedure as example 2.

Example 5

| | | |
|---|---|---|
| Flunarizine dihydrochloride: | | 5.9 mg |
| Sodium hydroxide | | 1.96 mg |
| Dist. water | | 2 mg |
| PEG 4000 | | 10 mg |
| Cremophor EL® | ad | 100 mg |

Same manufacturing as in example 2.

Example 6

| | | |
|---|---|---|
| Flunarizine dihydrochloride: | | 5.9 mg |
| Sodium hydroxide | | 1.96 mg |
| Dist. water | | 2 mg |
| Pluronic F68/F35® | | 25 mg |
| Cremophor EL® | ad | 100 mg |

Same manufacturing as in example 2.

Pharmacological examples

Example 7

The plasma concentrations after administration of 10 mg flunarizine were determined. The test formulations were administered orally by using capsules made according to example 1 and 2. At various time intervals blood samples were taken and the concentration of flunarizine in the blood plasma was determined by gas-chromatography.

| Time in hours | Plasma concentration of Flunarizine in ng/ml Formulation 1 | Plasma concentration of Flunarizine in ng/ml Formulation 2 |
|---|---|---|
| 0 | 0.00 | 0.00 |
| 0.5 | 3.00 | 10.32 |
| 1 | 10.28 | 26.20 |
| 2 | 20.83 | 37.69 |
| 3 | 21.11 | 34.55 |
| 4 | 20.20 | 29.61 |
| 6 | 16.98 | 23.66 |
| 8 | 14.68 | 17.26 |
| 24 | 3.11 | 4.39 |
| 32 | 2.67 | 3.58 |
| 48 | 1.93 | 2.64 |
| 72 | 1.35 | 1.90 |
| 96 | 0.78 | 1.52 |

Example 8

The plasma concentrations resulting from an oral intake of a reference formulation (formulation 1 made as shown in example 1) and a formulation according to the present invention (formulation 2 made according to example 2) in normal subjects and in subjects receiving a combined bicarbonate/ranitidine medication (reduced gastric acidity) were measured at various time intervals as in example 10. In the following table there are gathered a number of parameters deducted from results of these tests. In this table "normal" refers to normal subjects and "reduced" to subjects with reduced gastric acidity. $T_{max}$ is that point of time where the plasma concentration was at its maximum. $C_{max}$/g a.i. is the plasma concentration per gram active ingredient administered at the $T_{max}$ · $AUC_{(0-96h)}$/g a.i. gives the area under the curve per gram active ingredient in the time period from 0 to 96 hours after administration. $F_{rel}$ is the relative biological availability whereby the $AUC_{(0-96h)}$/g a.i. ingredient for the reference formulation is taken as 100.

| Parameter | normal 2x5 mg form. 1 | reduced 2x5 mg form. 1 | normal 2x3 mg form. 2 | reduced 2x3 mg form. 2 |
|---|---|---|---|---|
| T max | 2.8 | 5.8 | 3.4 | 3.1 |
| $C_{max}$/g a.i. (ng/ml.mg) | 2.82 | 1.87 | 3.31 | 3.21 |
| AUC(0-96 h)/g a.i. | 49 | 39.8 | 56.5 | 56.33 |
| $F_{rel}$ | 100 | 100 | 115 | 145 |

## Claims

1. A pharmaceutical composition containing polyethoxylated castor oil or hydrogenated castor oil obtained by reaction of 1 mole of the said oil with 5 to 100 moles of ethylene oxide, and as active ingredient a chemical compound of formula

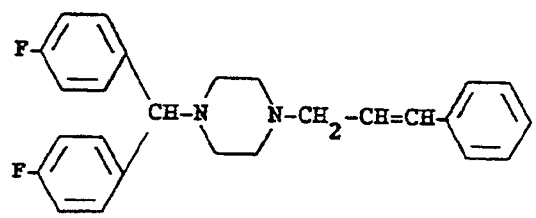

(I),

2. A composition according to claim 1 wherein the carrier is polyethoxylated castor oil or hydrogenated castor oil obtained by reaction of 1 mole of the said oil with 30 to 50 moles of ethylene oxide.

3. A composition according to claim 2 wherein the carrier is polyethoxylated castor oil or hydrogenated castor oil obtained by reaction of 1 mole of the said oil with about 40 moles of ethylene oxide.

4. A composition according to any of claims 1 to 3 containing over 50% of the said carrier.

5. A composition according to any of claims 1 to 4 containing over 80% of the said carrier.

6. A composition according to any of claims 1 to 5 wherein said composition is filled in a hard gelatin capsule.

7. A composition according to claim 6 wherein said composition contains from 0.1 to 5% by weight water and optionally from 0.1 to 3% by weight sodium hydroxide.

8. A process for preparing a composition as claimed in any of claims 1 to 7, characterized in that the active ingredient is intimately mixed with the carrier and the possible other adjuvants.

9. A process for preparing a composition as claimed in any of claims 1 to 8 characterized in that the components are intimately mixed at a temperature exceeding the melting point or weakening point of the said carrier.

10. A process according to claim 9 wherein the components are mixed in a heat-jacketed vessel at 45-50°C.

## Patentansprüche

1. Pharmazeutische Zusammensetzung, enthaltend polyethoxyliertes Rizinusöl oder hydriertes Rizinusöl, erhalten durch Umsetzung von 1 Mol des Öls mit 5 bis 100 Mol Ethylenoxid, und als wirksamen Bestandteil eine chemische Verbindung der Formel :

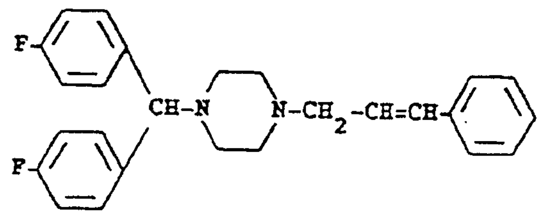

(I),

2. Zusammensetzung nach Anspruch 1, worin der Träger polyethoxyliertes Rizinusöl oder hydriertes Rizinusöl ist, das durch Umsetzung von 1 Mol des Öls mit 30 bis 50 Mol Ethylenoxid erhalten worden ist.

3. Zusammensetzung nach Anspruch 2, worin der Träger polyethoxyliertes Rizinusöl oder hydriertes Rizinusöl ist, das durch Umsetzung von 1 Mol des Öls mit etwa 40 Mol Ethylenoxid erhalten worden ist.

4. Zusammensetzung nach einem der Ansprüche 1 bis 3 mit einem Gehalt an über 50% des Trägers.

5. Zusammensetzung nach einem der Ansprüche 1 bis 4 mit einem Gehalt an über 80% des Trägers.

6. Zusammensetzung nach einem der Ansprüche 1 bis 5, worin die Zusammesetzung in eine Hartgelatinekapsel eingefüllt ist.

7. Zusammensetzung nach Anspruch 6, worin die Zusammensetzung 0,1 bis 5 Gew.-% Wasser und gegebenenfalls 0,1 bis 3 Gew.-% Natriumhydroxid enthält.

8. Verfahren zur Herstellung einer Zusammensetzung nach einem der Ansprüche 1 bis 7, dadurch gekennzeichnet, daß der wirksame Bestandteil innig mit dem Träger und den fakultativen weiteren Hilfsstoffen vermischt wird.

9. Verfahren zur Herstellung einer Zusammensetzung nach einem der Ansprüche 1 bis 8, dadurch gekennzeichnet, daß die Komponenten innig bei einer den Schmelz- oder Erweichungspunkt des Trägers übersteigenden Temperatur gemischt werden.

10.Verfahren nach Anspruch 9, worin die Komponenten in einem Behälter mit Wärmemantel bei 45 bis 50°C gemischt werden.

## Revendications

1. Composition pharmaceutique contenant de l'huile de ricin polyéthoxylée ou de l'huile de ricin hydrogénée polyéthoxylée obtenue par réaction d'1 mole de ladite huile avec de 5 à 100 moles d'oxyde d'éthylène, et comme ingrédient actif un composé chimique de formule

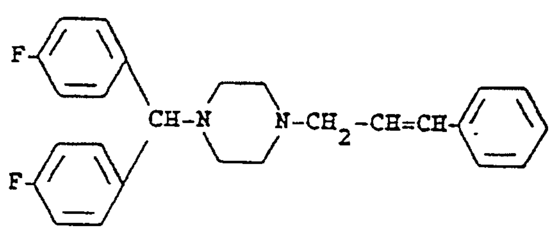

(I),

2. Composition selon la revendication 1, dans laquelle le support est de l'huile de ricin polyéthoxylée ou de l'huile de ricin hydrogénée polyéthoxylée obtenue par réaction d'1 mole de ladite huile avec de 30 à 50 moles d'oxyde d'éthylène.

3. Composition selon la revendication 2, dans laquelle le support est de l'huile de ricin polyéthoxylée ou de l'huile de ricin hydrogénée polyéthoxylée obtenue par réaction d'1 mole de ladite huile avec environ 40 moles d'oxyde d'éthylène.

4. Composition selon l'une quelconque des revendications 1 à 3 contenant plus de 50% dudit support.

5. Composition selon l'une quelconque des revendications 1 à 4 contenant plus de 80% dudit support.

6. Composition selon l'une quelconque des revendications 1 à 5, où ladite composition remplit une capsule de gélatine dure.

7. Composition selon la revendication 6, où ladite composition contient de 0,1 à 5% en poids d'eau et éventuellement de 0,1 à 3% en poids d'hydroxyde de sodium.

8. Procédé pour préparer une composition telle que revendiquée dans l'une quelconque des revendications 1 à 7, caractérisé en ce que l'ingrédient actif est mélangé de façon intime avec le support et les autres adjuvants

éventuels.

9. Procédé pour préparer une composition telle que revendiquée dans l'une quelconque des revendications 1 à 8, caractérisé en ce que les composants sont mélangés de façon intime à une température qui excède le point de fusion ou le point d'affaiblissement dudit support.

10. Procédé selon la revendication 9, dans lequel les composants sont mélangés dans un récipient à gaine chauffante, à 45-50°C.